# EUROPEAN PATENT SPECIFICATION

(11) **EP 2 818 199 B1**
(45) Date of publication and mention of the grant of the patent: **07.09.2022**
(21) Application number: 14166027.4
(22) Date of filing: 25.04.2014
(51) Int. Cl.: A61N 1/36, A61N 1/378

(54) **Implantable vagal neurostimulator with bounded autotitration**
Implantierbarer vagaler Neurostimulator mit beschränkter Selbsttitration
Neurostimulateur vagal implantable à auto-titrage limité

(30) Priority: 30.06.2013 US 201313931961
(43) Date of publication of application: 31.12.2014
(73) Proprietor: Cyberonics, Inc., Houston TX 77058 (US)
(72) Inventor: Libbus, Imad, Houston, TX Texas 77058 (US); Amurthur, Badri, Houston, TX Texas 77058 (US); Kenknight, Bruce H., Houston, TX Texas 77058 (US)
(74) Representative: Grünecker Patent- und Rechtsanwälte PartG mbB

(56) References cited:
- US-A- 5 215 086
- US-A1- 2008 033 509
- US-A1- 2008 300 654
- US-A1- 2011 313 488

## Description

### FIELD

This application relates in general to chronic cardiac dysfunction therapy and, in particular, to an implantable neurostimulator for providing electrical stimulation of cervical vagus nerves for treatment of chronic cardiac dysfunction with bounded titration.

### BACKGROUND

Vagal nerve stimulation (VNS), therapeutic electrical stimulation of a patient's cervical vagus nerve, is used for treatment of multiple health conditions, including clinical treatment of drug-refractory epilepsy and depression. VNS has also been proposed for therapeutic treatment of heart conditions such as Congestive Heart Failure (CHF), a chronic medical condition in which the heart is unable to pump sufficient blood to meet the body's needs. For instance, VNS has been demonstrated in canine studies as efficacious in simulated treatment of atrial fibrillation (AF) and heart failure, such as described in Zhang et al., "Therapeutic Effects of Selective Atrioventricular Node Vagal Stimulation in Atrial Fibrillation and Heart Failure," J. Cardiovasc. Electrophysiol., Vol. 24, pp. 86-91 (January 2013), the disclosure of which is incorporated by reference.

VNS therapy commonly requires an implantation of a neurostimulator, a surgery requiring several weeks of recovery before a patient can start receiving VNS therapy. Even after the recovery, the neurostimulator does not immediately start delivering a full therapeutic dose of VNS to avoid causing significant patient discomfort and other undesirable side effects. Instead, to allow the patient to adjust to the VNS therapy, the intensity is gradually up titrated over a period of time under a control of a physician. Medically, such a titration can be completed in a period not exceeding two months. The titration can take significantly longer in practice because the increase in intensity must generally be performed by a physician or another healthcare professional. Thus, for every step in the titration to take place, the patient has to visit the physician's office. These visits generally occur once every one to three months due to scheduling conflicts, and can extend the titration process to as much as a year, during which the patient in need of VNS does not receive the VNS at the full therapeutic intensity.

The medically unnecessary delays in delivering therapeutic levels of VNS can further accumulate if the therapy is disrupted following the initial titration, such as if the implanted VNS neurostimulator runs out of power. Power for a conventional VNS neurostimulator is typically provided by an onboard battery. Delivering therapeutic VNS through an implantable neurostimulator presents battery longevity concerns similar to other types of implantable pulse generators, although a VNS neurostimulator is therapeutic and non-life sustaining. Still, as the duty cycle of a VNS neurostimulator can at times be near constant, battery depletion can occur at a faster rate than with cardiac pacemakers, implantable cardioverter defibrillators (ICDs) and similar implantable devices that are triggered relatively infrequently under normal conditions. The in-service lifetime of a conventional VNS neurostimulator typically varies from three to seven years, depending upon programming, particularly duty cycle and stimulation intensity. As well, an increase in lead impedance can further cause premature battery depletion. Battery depletion necessitates eventual neurostimulator explantation and replacement, with attendant surgical risks of injury and infection. The disruption in therapy caused by the surgery requires a new cycle of titration of therapeutic intensity, which as described above, can take up to a year. Thus, a patient using a conventional VNS stimulator can be subjected to repeated, prolonged, and medically unjustified delays in receiving VNS stimulation that can be harmful to the patient's health.

In addition to the challenges associated with conventional VNS stimulators described above, conventional VNS stimulators do not achieve optimum effectiveness in treating the underlying causes of CHF. CHF, as well as other forms of chronic cardiac dysfunction, are generally attributed to an autonomic imbalance of the sympathetic and parasympathetic nervous systems that, if left untreated, can lead to cardiac arrhythmogenesis, progressively worsening cardiac function and eventual patient death. Furthermore, CHF is pathologically characterized by an elevated neuroexitatory state and is accompanied by physiological indications of impaired arterial and cardiopulmonary baroreflex function with reduced vagal activity.

Experimental VNS for cardiac therapy has focused on the efferent nerves of the parasympathetic nervous system, such as described in Sabbah et al., "Vagus Nerve Stimulation in Experimental Heart Failure," Heart Fail. Rev., 16:171-178 (2011). Sabbah discusses canine studies using a VNS system manufactured by BioControl Medical Ltd., Yehud, Israel, that includes an electrical pulse generator, right ventricular endocardial sensing lead, and right vagus nerve cuff stimulation lead. The sensing lead enables closed loop synchronization to the cardiac cycle; stimulation is delivered only when heart rate increases above a preset threshold. An asymmetric tri-polar nerve cuff electrode provides cathodic induction of action potentials while simultaneously applying asymmetric anodal blocks that lead to preferential activation of vagal efferent fibers. Stimulation is provided at an intensity and frequency intended to measurably reduce basal heart rate by ten percent by preferential stimulation of efferent vagus nerve fibers leading to the heart while blocking afferent neural impulses to the brain. The degree of therapeutic effect on parasympathetic activation occurs through incidental recruitment of afferent parasympathetic nerve fibers in the vagus, as well as through recruitment of efferent fibers.

Other uses of electrical nerve stimulation for therapeutic treatment of cardiac and physiological conditions are described. For instance, U.S. Patent No. 8,219,188, issued July 10, 2012 to Craig discloses synchronization of vagus nerve stimulation with a physiological cycle, such as the cardiac or respiratory cycle, of a patient. Electrical stimulation is applied to the vagus nerve at a selected point in the physiological cycle correlated with increased afferent conduction, such as a point from about 10 msec to about 800 msec after an R-wave of the patient's ECG, optionally during inspiration by the patient; to increase heart rate variability, such as a point from about 10 msec to about 800 msec after an R-wave of the patient's ECG, optionally during expiration by the patient; not correlated with increased efferent conduction on the vagus nerve; to generate efferent electrical activity on the vagus nerve; or upon the detection of a symptom of a medical condition. In a further embodiment, conventional VNS is applied to the vagus nerve along with microburst electrical signals, which is a portion of a therapeutic electrical signal having a limited plurality of pulses, separated from one another by interpulse intervals, and a limited burst duration, separated from one another by interburst periods. Stimulation may be applied to generate efferent electrical activity on the nerve in a direction away from the central nervous system; through a "blocking" type of electrical signal, such that both afferent and efferent electrical activity on the nerve is prevented from traveling further; or wherein afferent fibers are stimulated while efferent fibers are not stimulated or are blocked, and vice versa. By applying a series of microbursts to the vagus nerve, enhanced vagal evoked potentials (eVEP) are produced in therapeutically significant areas of the brain, in contrast to conventional VNS alone, which fails to produce eVEP.

U.S. Patent No. 6,600,954, issued July 29, 2003 to Cohen et al. discloses a method and apparatus for selective control of nerve fiber activations for reducing pain sensations in the legs and arms. An electrode device is applied to a nerve bundle capable of generating, upon activation, unidirectional action potentials that propagate through both small diameter and large diameter sensory fibers in the nerve bundle, and away from the central nervous system.

U.S. Patent No. 6,684,105, issued January 27, 2004 to Cohen et al. discloses an apparatus for treatment of disorders by unidirectional nerve stimulation. An apparatus for treating a specific condition includes a set of one or more electrode devices that are applied to selected sites of the central or peripheral nervous system of the patient. For some applications, a signal is applied to a nerve, such as the vagus nerve, to stimulate efferent fibers and treat motility disorders, or to a portion of the vagus nerve innervating the stomach to produce a sensation of satiety or hunger. For other applications, a signal is applied to the vagus nerve to modulate electrical activity in the brain and rouse a comatose patient, or to treat epilepsy and involuntary movement disorders.

U.S. Patent No. 7,123,961, issued October 17, 2006 to Kroll et al. discloses stimulation of autonomic nerves. An autonomic nerve is stimulated to affect cardiac function using a stimulation device in electrical communication with the heart by way of three leads suitable for delivering multi-chamber stimulation and shock therapy. For arrhythmia detection, the device utilizes atrial and ventricular sensing circuits to sense cardiac signals to determine whether a rhythm is physiologic or pathologic. The timing intervals between sensed events are classified by comparing them to a predefined rate zone limit and other characteristics to determine the type of remedial therapy needed, which includes bradycardia pacing, anti-tachycardia pacing, cardioversion shocks (synchronized with an R-wave), or defibrillation shocks (delivered asynchronously).

U.S. Patent No. 7,225,017, issued May 29, 2007 to Shelchuk discloses terminating ventricular tachycardia in connection with any stimulation device that is configured or configurable to stimulate nerves, or stimulate and shock a patient's heart. Parasympathetic stimulation is used to augment anti-tachycardia pacing, cardioversion, or defibrillation therapy. To sense atrial or ventricular cardiac signals and provide chamber pacing therapy, particularly on the left side of the heart, the stimulation device is coupled to a lead designed for placement in the coronary sinus or its tributary veins. Cardioversion stimulation is delivered to a parasympathetic pathway upon detecting a ventricular tachycardia. A stimulation pulse is delivered via the lead to electrodes positioned proximate to the parasympathetic pathway according to stimulation pulse parameters based at least in part on the probability of reinitiation of an arrhythmia.

U.S. Patent No. 7,277,761, issued October 2, 2007 to Shelchuk discloses vagal stimulation for improving cardiac function in heart failure patients. An autonomic nerve is stimulated to affect cardiac function by way of three leads suitable for delivering multi-chamber endocardial stimulation and shock therapy. When the stimulation device is intended to operate as an implantable cardioverter-defibrillator (ICD), the device detects the occurrence of an arrhythmia, and applies a therapy to the heart aimed at terminating the detected arrhythmia. Defibrillation shocks are generally of moderate to high energy level, delivered asynchronously, and pertaining exclusively to the treatment of fibrillation.

U.S. Patent No. 7,295,881, issued November 13, 2007 to Cohen et al. discloses nerve branch-specific action potential activation, inhibition and monitoring. Two preferably unidirectional electrode configurations flank a nerve junction from which a preselected nerve branch issues with respect to the brain. Selective nerve branch stimulation can be used in conjunction with nerve-branch specific stimulation to achieve selective stimulation of a specific range of fiber diameters, substantially restricted to a preselected nerve branch, including heart rate control, where activating only the vagal B nerve fibers in the heart, and not vagal A nerve fibers that innervate other muscles, can be desirous.

U.S. Patent No. 7,778,703, issued August 17, 2010 to Gross et al. discloses selective nerve fiber stimulation for treating heart conditions. An electrode device is coupled to a vagus nerve and a control unit applies stimulating and inhibiting currents to the vagus nerve, which are capable of respectively inducing action potentials in a therapeutic direction in first and second sets of nerve fibers in the vagus nerve and inhibiting action potentials in the therapeutic direction in the second set of nerve fibers only. The nerve fibers in the second set have larger diameters than the first set's nerve fibers. Typically, the system is configured to treat heart failure or heart arrhythmia, such as AF or tachycardia by slowing or stabilizing the heart rate, or reducing cardiac contractility.

U.S. Patent No. 7,813,805, issued October 12, 2010 to Farazi and U.S. Patent No. 7,869,869, issued January 11, 2011 to Farazi both disclose subcardiac threshold vagus nerve stimulation. A vagus nerve stimulator is configured to generate electrical pulses below a cardiac threshold, which are transmitted to a vagus nerve, so as to inhibit or reduce injury resulting from ischemia. For arrhythmia detection, a heart stimulator utilizes atrial and ventricular sensing circuits to sense cardiac signals to determine whether a rhythm is physiologic or pathologic. In low-energy cardioversion, an ICD device typically delivers a cardioversion stimulus synchronously with a QRS complex. If anti-tachycardia pacing or cardioversion fails to terminate a tachycardia, after a programmed time interval or if the tachycardia accelerates, the ICD device initiates defibrillation.

U.S. Patent App. Pub. No. 2010/0331908, filed September 10, 2010 by Farazi discloses subcardiac threshold vagus nerve stimulation in which a vagal nerve stimulator generates electrical pulses below a cardiac threshold of the heart for treating an ischemia of the heart, or for reducing a defibrillation threshold of the heart. The cardiac threshold is a threshold for energy delivered to the heart above which there is a slowing of the heart rate or the conduction velocity. In operation, the vagal nerve stimulator generates the electrical pulses below the cardiac threshold, that is, subcardiac threshold electrical pulses, such that the beat rate of the heart is not affected. Although the function of the vagal nerve stimulator is to treat an ischemia, or to reduce a defibrillation threshold of the heart, in other embodiments, the vagal nerve stimulator may function to treat heart failure, reduce an inflammatory response during a medical procedure, stimulate the release of insulin for treating diabetes, suppress insulin resistance for treating diabetes, or treat an infarction of the heart.

U.S. Patent No. 7,634,317, issued December 15, 2009, to Ben-David et al. discloses techniques for applying, calibrating and controlling nerve fiber stimulation, which includes a vagal stimulation system comprising a multipolar electrode device that is applied to a portion of a vagus nerve (a left vagus nerve and/or right vagus nerve), which innervates a heart of a subject. Alternatively, the electrode device is applied to an epicardial fat pad, a pulmonary vein, a carotid artery, a carotid sinus, a coronary sinus, a vena cava vein, a right ventricle, or a jugular vein. The system is utilized for treating a heart condition such as heart failure and/or cardiac arrhythmia; the vagal stimulation system further comprises an implantable or external control unit, which typically communicates with electrode device over a set of leads. Typically, the control unit drives the electrode device to (i) apply signals to induce the propagation of efferent nerve impulses towards heart, and (ii) suppress artificially-induced afferent nerve impulses towards a brain of the subject, to minimize unintended side effects of the signal application; the efferent nerve pulses in vagus nerve are typically induced by the electrode device to regulate the heart rate of the subject.

Finally, U.S. Patent No. 7,885,709, issued February 8, 2011 to Ben-David discloses nerve stimulation for treating disorders. An electrode device stimulates the vagus nerve, so as to modify heart rate variability, or to reduce heart rate, by suppressing the adrenergic (sympathetic) system. Typically, the system is configured to treat heart failure or heart arrhythmia. Therapeutic effects of reduction in heart rate variability include the narrowing of the heart rate range, thereby eliminating very slow heart rates and very fast heart rates. For this therapeutic application, the control unit is typically configured to reduce low-frequency heart rate variability, and to adjust the level of stimulation applied based on the circadian and activity cycles of the subject. Therapeutic effects also include maximizing the mechanical efficiency of the heart by maintaining relatively constant ventricular filling times and pressures.

US 2011/313488 A1 discloses a system for providing electrical stimulation with a patient tolerance input, which may trigger a titration sweep. A titration sweep is performed for selecting stimulation parameters, including the step of stepping up the amplitude and determining whether the safety limit has been met, thereby confirming that the charge per phase and charge density per phase does not violate the safety limit.

Notwithstanding, a need remains for an approach to therapeutically treating chronic cardiac dysfunction through a neurostimulator that allows to avoid medically unjustified delays in VNS therapy associated with titration of intensity of VNS stimulation.

### SUMMARY

Excessive sustained activation of the sympathetic nervous system has a deleterious effect on long term cardiac performance and on the quality of life and survival of patients suffering from chronic cardiac dysfunction. CHF patients are at increased risk of atrial tachyarrhythmias, such as AF and atrial flutter, and ventricular tachyarrhythmias, such as ventricular tachycardia (VT) and ventricular fibrillation (VF)), particularly when the underlying morbidity is a form of coronary artery disease, cardiomyopathy, mitral valve prolapse, or other valvular heart disease. Low intensity peripheral neurostimulation therapies that target the imbalance of the autonomic nervous system have been shown to improve clinical outcomes. Thus, bi-directional autonomic regulation therapy is delivered to the cervical vagus nerve at an intensity that is insufficient to elicit pathological or acute physiological side effects and without the requirement of an enabling physiological feature or triggering physiological marker. Letting a patient in need of such therapy to control the therapy titration following the implantation of a VNS neurostimulator allows the patient to receive VNS stimulation at a full therapeutic intensity sooner than if the titration is performed entirely under a control of a healthcare professional. Furthermore, placing physician-defined boundaries on the titration mitigates the risks associated with over-aggressive patient titration of the stimulation.

One embodiment provides a system for providing electrical stimulation of cervical vagus nerves for treatment of chronic cardiac dysfunction with bounded titration. The system includes a patient-operable external controller configured to transmit a plurality of unique signals including a unique signal associated with an up-titration command. The system further includes an implantable neurostimulator. The implantable neurostimulator includes a pulse generator configured to deliver electrical therapeutic stimulation tuned to restore autonomic balance through continuously-cycling, intermittent and periodic electrical pulses simultaneously delivered at an intensity that avoids acute physiological side effects and with an unchanging cycle not triggered by physiological markers in a manner that results in creation and propagation (in both afferent and efferent directions) of action potentials within neuronal fibers comprising a cervical vagus nerve of a patient through a pair of helical electrodes via an electrically coupled nerve stimulation therapy lead. The implantable neurostimulator also includes a recordable memory storing an autotitration operating mode that includes a maximum stimulation intensity and is configured to increase an intensity of the delivered electrical therapeutic stimulation up to a level not exceeding the maximum stimulation intensity upon receipt of the unique signal associated with the up-titration command.

A further embodiment provides an implantable neurostimulator-implemented method, not according to the invention and present for illustration purposes only, for providing electrical stimulation of cervical vagus nerves for treatment of chronic cardiac dysfunction with bounded titration. An implantable neurostimulator that includes a pulse generator configured to deliver electrical therapeutic stimulation in a manner that results in creation and propagation (simultaneously in both afferent and efferent directions) of action potentials within neuronal fibers comprising the cervical vagus nerve of a patient is provided. An autotitration operating mode is stored into a recordable memory, which includes: parametrically defining a maximum stimulation intensity of the electrical therapeutic stimulation deliverable under the autotitration operating mode; and parametrically defining a titration dose of the electrical therapeutic stimulation tuned to restore cardiac autonomous balance through continuously-cycling, intermittent and periodic electrical pulses at an intensity not exceeding the maximum stimulation intensity that avoids acute physiological side affects and with an unchanging cycle not triggered by physiological markers. The titration dose is therapeutically delivered to the vagus nerve independent of cardiac cycle via the pulse generator in the implantable neurostimulator through at least a pair of helical electrodes electrically coupled to the pulse generator via a nerve stimulation therapy lead. Upon receiving a remotely applied signal uniquely associated with an up-titration command from a patient-operable external controller, the titration dose intensity is increased to a level not exceeding the maximum stimulation intensity.

A still further embodiment provides a system for providing a bounded titration of electrical stimulation of cervical vagus nerves for treatment of chronic cardiac dysfunction following the stimulation start or disruption. The system includes a patient-operable external controller configured to transmit a unique signal associated with an up-titration command. The system further includes an implantable neurostimulator powered by a rechargeable battery in included in the neurostimulator. The neurostimulator includes a pulse generator configured to deliver electrical therapeutic stimulation tuned to restore autonomic balance through continuously-cycling, intermittent and periodic electrical pulses simultaneously delivered at an intensity that avoids acute physiological side effects and with an unchanging cycle not triggered by physiological markers in a manner that results in creation and propagation (in both afferent and efferent directions) of action potentials within neuronal fibers comprising a cervical vagus nerve of a patient through a pair of helical electrodes via an electrically coupled nerve stimulation therapy lead. The neurostimulator also includes a recordable memory storing an autotitration operating mode that includes a maximum stimulation intensity and that is configured to perform a stimulation autotitration following at least one of the neurostimulator's implantation into the patient and a disruption of the delivery of the electrical therapeutic stimulation caused by a depletion of the neurostimulator's power, the autotitration including an increase in intensity of the delivered electrical therapeutic stimulation, to a level not exceeding the maximum stimulation intensity, performed upon receipt of the unique signal associated with the up-titration command. The neurostimulator further includes an energy receiver configured to transcutaneously receive energy and recharge the rechargeable battery with the received energy.

By restoring autonomic balance, therapeutic VNS operates acutely to decrease heart rate, increase heart rate variability and coronary flow, reduce cardiac workload through vasodilation, and improve left ventricular relaxation. Over the long term, VNS provides the chronic benefits of decreased negative cytokine production, increased baroreflex sensitivity, increased respiratory gas exchange efficiency, favorable gene expression, renin-angiotensin-aldosterone system down-regulation, and anti-arrhythmic, anti-apoptotic, and ectopy-reducing anti-inflammatory effects. Furthermore, patient-controlled autotitration of VNS stimulation allows the patient to avoid the unnecessary delays associated with visiting a physician's office for every step in the titration to take place. Furthermore, providing the neurostimulator with a rechargeable battery allows to lessen the frequency of a disruption in therapy and the associated need to retitrate the therapy following a replacement of a neurostimulator with a depleted battery.

Still other embodiments of the present invention will become readily apparent to those skilled in the art from the following detailed description, wherein are described embodiments by way of illustrating the best mode contemplated for carrying out the invention.

Accordingly, the drawings and detailed description are to be regarded as illustrative in nature and not as restrictive.

### BRIEF DESCRIPTION OF THE DRAWINGS

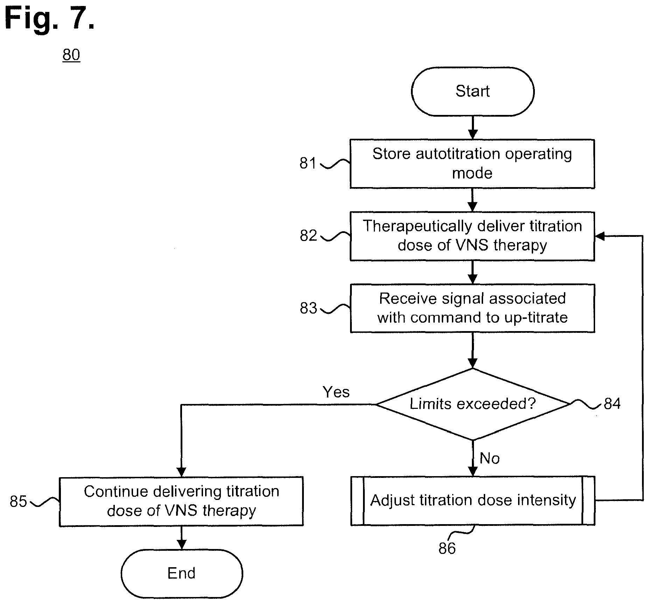

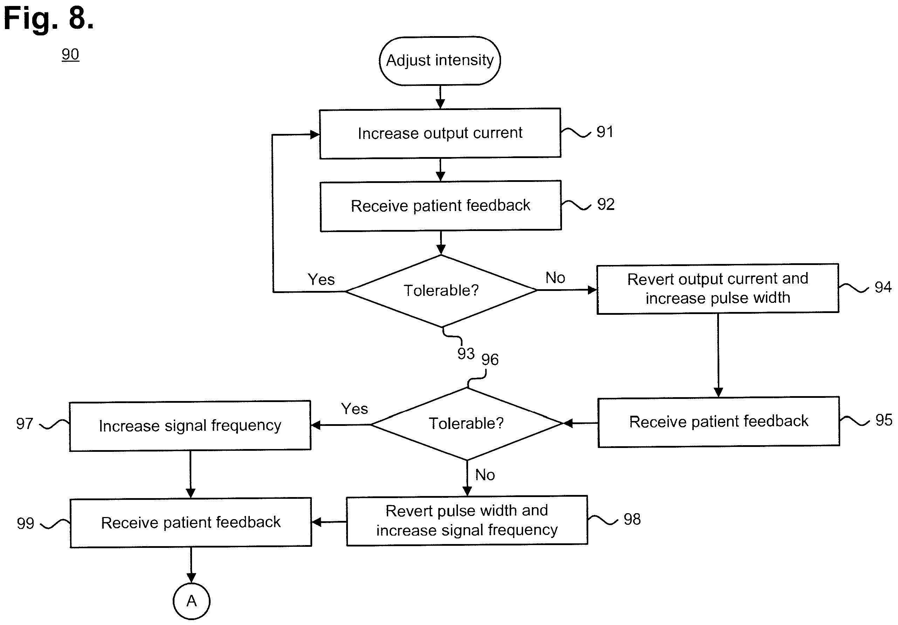

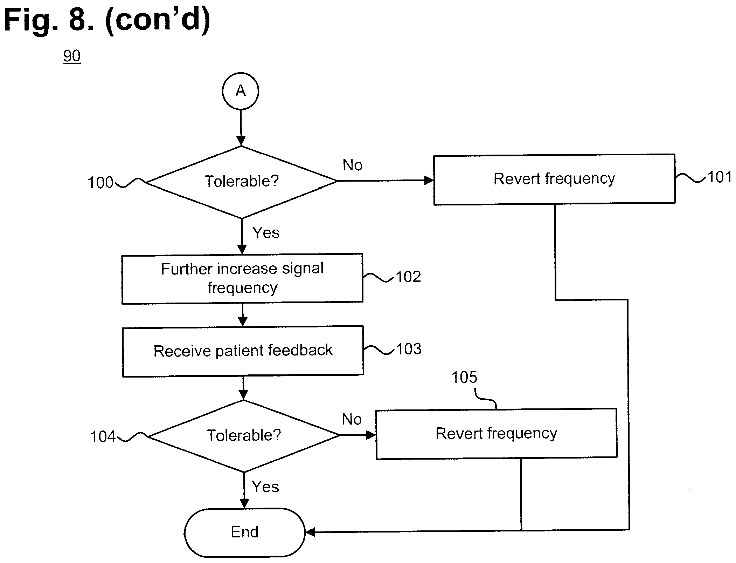

The following method and routine shown in Figures 7 and 8 are not according to the invention and are present for illustration purposes only.
FIGURE 1 is a front anatomical diagram showing, by way of example, placement of an implantable vagus stimulation device in a male patient, in accordance with one embodiment.
FIGURES 2A and 2B are diagrams respectively showing the rechargeable implantable neurostimulator for treatment of chronic cardiac dysfunction and the simulation therapy lead of FIGURE I.
FIGURE 3 is a diagram showing the charging components of the implantable neurostimulator of FIGURE 1.
FIGURE 4 is a diagram showing an external charger for use with the implantable neurostimulator of FIGURE 1.
FIGURE 5 is a graph showing, by way of example, the relationship between the targeted therapeutic efficacy and the extent of potential side effects resulting from use of the implantable neurostimulator of FIGURE 1.
FIGURE 6 is a timing diagram showing, by way of example, a stimulation cycle and an inhibition cycle of VNS as provided by implantable neurostimulator of FIGURE 1.
FIGURE 7 is a flow diagram showing an implantable neurostimulator-implemented method for treatment of chronic cardiac dysfunction with bounded titration in accordance with one embodiment.
FIGURE 8 is a flow diagram showing a routine for adjusting intensity of VNS therapy during autotitration based on patient feedback for the method of FIGURE 7.

### DETAILED DESCRIPTION

Changes in autonomic control of the cardiovascular systems of patients suffering from CHF and other cardiovascular diseases push the autonomic nervous system out of balance and favor increased sympathetic and decreased parasympathetic central outflow. The imbalance is accompanied by pronounced elevation of basal heart rate arising from chronic sympathetic hyperactivation along the neurocardiac axis. Medically unnecessary delays in providing CHF therapy to a patient can further deteriorate the patient's health.

Low intensity peripheral neurostimulation therapies that target the imbalance of the autonomic nervous system have been shown to improve clinical outcomes in patients treated for three to twelve months. Bi-directional autonomic regulation therapy results in simultaneous creation and propagation of efferent and afferent action potentials within the vagus nerve. In contrast to conventional approaches to VNS, the neurostimulation is delivered bi-directionally and at an intensity that is insufficient to elicit pathological or acute physiological side effects, such as acute cardiac arrhythmias, and without the requirement of an enabling physiological feature or triggering physiological marker, such as heart rate or heart rate variability (HRV), such as for purposes of timing stimulation delivery, to confirm therapeutic effect, or other ends. Here, upon continuously-cycling, intermittent and periodic low intensity stimulation of the cervical vagus nerve, action potentials propagate away from the stimulation site in two directions, efferently toward the heart to influence the intrinsic cardiac nervous system and the heart and afferently toward the brain to influence central elements of the nervous system.

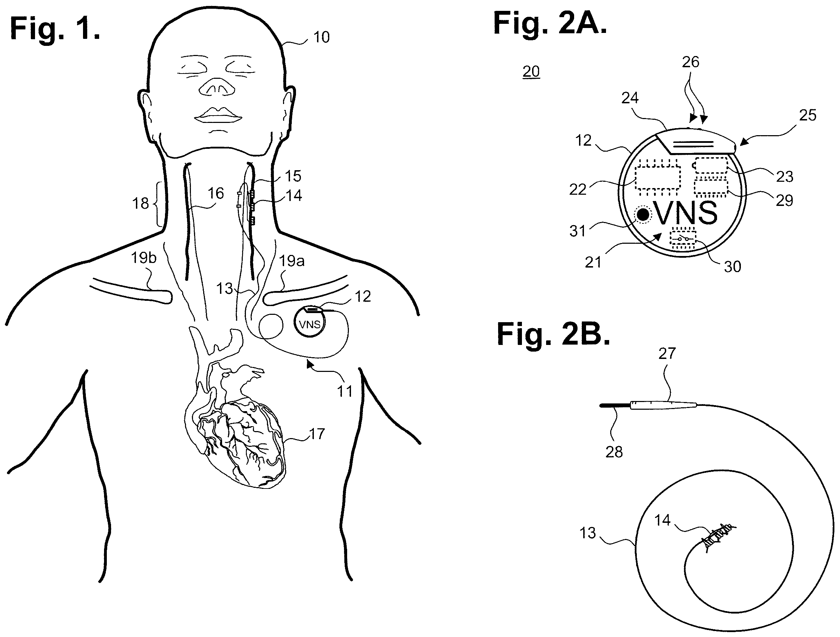

An implantable vagus nerve stimulator (neurostimulator), such as used to treat drug-refractory epilepsy and depression, can be adapted for use in managing chronic cardiac dysfunction through therapeutic bi-directional vagal stimulation. While the VNS therapy may not be delivered immediately at a full therapeutic intensity, allowing the patient to control the titration of intensity of VNS stimulation delivered to the patient makes this titration process ("autotitration") significantly faster when compared to a titration entirely controlled by a physician. In particular, the intensity of VNS delivered by the neurostimulator can be adjusted by a patient-operable external controller, which allows the patient to up-titrate the VNS within bounds set by in advance by a physician or another healthcare professional. Thus, by allowing the patient to perform up-titration of VNS intensity, the number of visits to a physician's office is reduced, which in turn reduces the length of the titration process. Furthermore, the neurostimulator can also be inductively charged *in situ* to counter battery depletion during operation, and preventing the need to perform an additional cycle of titration necessary when the neurostimulator is surgically replaced due to a depleted battery. FIGURE 1 is a front anatomical diagram showing, by way of example, placement of an implantable vagus nerve stimulation (VNS) device 11 in a male patient 10, in accordance with one embodiment. The VNS provided through the stimulation device 11 operates under several mechanisms of action. These mechanisms include increasing parasympathetic outflow and inhibiting sympathetic effects by blocking norepinephrine release. More importantly, VNS triggers the release of acetylcholine (ACh) into the synaptic cleft, which has beneficial anti-arrhythmic, anti-apoptotic, and ectopy-reducing anti-inflammatory effects.

The implantable vagus stimulation device 11 includes at least three implanted components, an implantable neurostimulator 12, a therapy lead 13, and helical electrodes 14. The implantable vagus stimulation device 11 can be remotely accessed following implantation through an external programmer (not shown) and patient-operable external controller (not shown). For example, the implantable vagus stimulation device 11 can be remotely accessed following implantation through the external programmer by which the neurostimulator 12 can be remotely checked and programmed. In particular, the external programmer can be used to set parameters for delivering VNS stimulation, such as duty cycle, output current (amplitude), frequency, and pulse width, with these parameters defining the intensity at which VNS is delivered. For example, the external programmer can be used to program a set of maximum stimulation parameters of VNS therapy that the neurostimulator 12 can deliver under any operating mode. For a commercial device sold by Cyberonics, Inc., Houston, TX, such parameters could be an output current of 3.5 MA, signal frequency of 30 Hz, and pulse width of 1000 µsec. Other parameters are possible.

The external programmer can also be used by a healthcare professional to store into the neurostimulator 12 an autotitration operating mode, described in detail *infra* with reference to FIGURES 7 and 8, which directs delivery of VNS during the autotitration process under a control of a patient-operable external controller. The autotitration operating mode includes limits, or boundaries, of the patient-controlled autotitration. For example, the autotitration operating mode can include a set of one or more maximum stimulation parameters defining the maximum stimulation intensity of VNS therapy that the neurostimulator can deliver during the autotitration process; this limit prevents the patient 10 from autotitrating above the maximum stimulation intensity. Once the maximum stimulation intensity is achieved during the autotitration process, further up-titration is prevented, and the patient 10 needs to visit a healthcare professional's office, where the healthcare professional can use the external programmer to program into the neurostimulator 12 an updated set of maximum stimulation parameters. Other limitations can be included in the operating mode. For example, a patient 10 may be limited to performing autotitration according to a predefined schedule, with the permissible frequency of patient-controlled increases in intensity and the period of time during which the patient can perform the autotitration being programmed into the operating mode. Other limits are possible. As described further *infra,* with reference to FIGURES 7 and 8, the autotitration operating mode further defines the increments at which the stimulation parameters are increased during the autotitration as well as the adjustments of the stimulation parameters that occur if the patient 10 indicates that an increased level of stimulation is not tolerable. Other aspects of the autotitration process can be addressed by the operating mode.

A patient-operable external controller (not shown) allows the patient to control the autotitration process, subject to the limits set by the healthcare professional in the autotitration operating mode. The patient-operable external controller is configured to control the neurostimulator 12 by transmitting to the neurostimulator 12 unique signals associated with commands, such as a command to increase (up-titrate) intensity of VNS stimulation, or patient feedback regarding whether an increased intensity of VNS therapy is tolerable. The patient-operable external controller can also suspend indefinitely VNS stimulation by supplying a magnetic signal to the neurostimulator, as further described in detail in the commonly-assigned U.S. Patent application, "Vagus Nerve Neurostimulator With Multiple Patient-Selectable Modes For Treating Chronic Cardiac Dysfunction," Serial No. 13/352,244, filed on January 17, 2012, pending, cited *supra* (the "13/352,244 application"). Other functionality of the patient-operable external controller is possible, as described in the 13/352,244 application cited *supra.*

In one embodiment, the patient-operable external controller can be an external magnet, such as described in commonly-assigned U.S. Patent application, entitled "Implantable Device For Facilitating Control Of Electrical Stimulation Of Cervical Vagus Nerves For Treatment Of Chronic Cardiac Dysfunction," Serial No. 13/314,130, filed on December 7, 2011, pending.

In a further embodiment, the patient-operable external controller can be an external electromagnetic controller, such as described in the 13/352,244 application cited *supra.* Other types of patient-operable external controllers are possible. Together, the implantable vagus stimulation device 11, the external programmer, and one or more of the external controllers described above can form a VNS therapeutic delivery system.

The neurostimulator 12 is implanted in the patient's right or left pectoral region generally on the same side (ipsilateral) as the cervical vagus nerve 15, 16 to be stimulated, although other neurostimulator-vagus nerve configurations, including contra-lateral and bi-lateral, are possible. The helical electrodes 14 are generally implanted on the vagus nerve 15, 16 about halfway between the clavicle 19a-b and the mastoid process. The therapy lead 13 and helical electrodes 14 are implanted by first exposing the carotid sheath and chosen vagus nerve 15, 16 through a latero-cervical incision on the ipsilateral side of the patient's neck 18. The helical electrodes 14 are then placed onto the exposed nerve sheath and tethered. A subcutaneous tunnel is formed between the respective implantation sites of the neurostimulator 12 and helical electrodes 14, through which the therapy lead 13 is guided to the neurostimulator 12 and securely connected.

The stimulation device 11 bi-directionally stimulates the vagus nerve 15, 16 through multimodal application of continuously-cycling, intermittent and periodic electrical stimuli, which are parametrically defined through stored stimulation parameters and timing cycles according to various operating modes. While the autotitration operating mode described *infra* with reference to FIGURES 7 and 8 allows titration of VNS intensity under a control of a patient-operable external controller, other operating modes can be implemented by the stimulation device 11. For example, in further embodiments, tachycardia and bradycardia in VNS-titrated patients can be respectively managed, such as described in commonly-assigned U.S. Patent Application, entitled "Implantable Neurostimulator-Implemented Method for Managing Tachyarrhythmia Through Vagus Nerve Stimulation," Serial No. 13,673,766, filed on November 9, 2012, pending, and U.S. Patent application, entitled "Implantable Neurostimulator-Implemented Method for Managing Bradycardia through Vagus Nerve Stimulation," Serial No. 13/554,656, filed on July 20, 2012, pending.

In a still further embodiment, prolonged activation of the sympathetic nervous system during post-exercise recovery periods, particularly in patients with CCD, can be managed through application of a "boost" dose of VNS, such as described in commonly-assigned U.S. Patent application, entitled "Implantable Neurostimulator-Implemented Method for Enhancing Post-Exercise Recovery through Vagus Nerve Stimulation," Serial No. 13/673,795, filed on November 9, 2012, pending.

Both sympathetic and parasympathetic nerve fibers are stimulated. Cervical vagus nerve stimulation results in the creation and propagation of action potentials (in both afferent and efferent directions) within neuronal fibers comprising the cervical vagus nerve from the site of stimulation to restore cardiac autonomic balance. Afferent action potentials propagate toward the parasympathetic nervous system's origin in the medulla in the nucleus ambiguus, nucleus tractus solitarius, and the dorsal motor nucleus, as well as towards the sympathetic nervous system's origin in the intermediolateral cell column of the spinal cord. Efferent action potentials propagate toward the heart 17 to activate the components of the heart's intrinsic nervous system. The helical electrodes 14 can be implanted on either the left or right vagus cervical nerve 15, 16. The right vagus nerve 16 has a moderately lower stimulation threshold than the left vagus nerve 15 for heart rate affects at the same parametric levels of VNS.

The VNS therapy is delivered autonomously to the patient's vagus nerve 15, 16 through three implanted components that include a neurostimulator 12, therapy lead 13, and helical electrodes 14. FIGURES 2A and 2B are diagrams respectively showing the implantable neurostimulator 12 and the stimulation therapy lead 13 of FIGURE 1. In one embodiment, the neurostimulator 12 can be adapted from a VNS Therapy AspireSR Model 105 or Model 106 pulse generator, manufactured and sold by Cyberonics, Inc., Houston, TX, although other manufactures and types of single-pin receptacle implantable VNS neurostimulators with or without integrated leadless heart rate sensors could also be used. The stimulation therapy lead 13 and helical electrodes 14 are generally fabricated as a combined assembly and can be adapted from a Model 302 lead, PerenniaDURA Model 303 lead, or PerenniaFLEX Model 304 lead, also manufactured and sold by Cyberonics, Inc., in two sizes based on helical electrode inner diameter, although other manufactures and types of single-pin receptacle-compatible therapy leads and electrodes could also be used.

Referring first to FIGURE 2A, the neurostimulator 12 provides multimodal vagal stimulation. The neurostimulator 12 includes an electrical pulse generator that is tuned to restore autonomic balance by triggering action potentials that propagate bi-directionally (both afferently and efferently) within the vagus nerve 15, 16. The neurostimulator 12 includes an electrical pulse generator that is tuned to restore autonomic balance by triggering the creation of action potentials that propagate both afferently and efferently within the vagus nerve 15, 16. The neurostimulator 12 is enclosed in a hermetically sealed housing 21 constructed of a biocompatible, implantation-safe material, such as titanium. The housing 21 contains electronic circuitry 22 powered by a primary rechargeable battery 23, which can be a lithium-ion battery, such as lithium nickel manganese cobalt oxide. The electronic circuitry 22 is implemented using complementary metal oxide semiconductor (CMOS) integrated circuits that include a microprocessor controller that executes a control program according to stored stimulation parameters and timing cycles; a voltage regulator that regulates system power; logic and control circuitry, including a recordable memory 29 within the operating modes and stimulation parameters are stored, that controls overall pulse generator function, receives and implements programming commands from the external programmer, or other external source, collects and stores telemetry information, processes sensory input, and controls scheduled and sensory-based therapy outputs; a transceiver that remotely communicates with the external programmer using radio frequency signals; an antenna, which receives programming instructions and transmits the telemetry information to the external programmer; and a reed switch 30 that provides remote access to the operation of the neurostimulator 12 using the external programmer or the patient-operable external controller. The recordable memory 29 can include both volatile (dynamic) and persistent (static) forms of memory, such as firmware within which the stimulation parameters and timing cycles can be stored. Other electronic circuitry and components are possible.

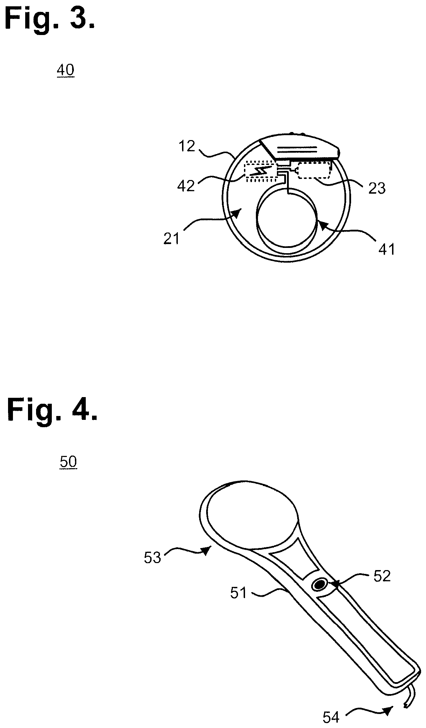

Power supplied to the neurostimulator 12 is provided by the rechargeable battery 23, which can be inductively charged by the patient 10 or caregiver as necessary to replenish the battery 23. Recharging the battery 23 allows delaying the disruption in delivery of VNS therapy caused by a depletion of the neurostimulator's 12 power, neurostimulator 12 explantation, and a subsequent need to perform another round of VNS titration. FIGURE 3 is a diagram showing the charging components of the implantable neurostimulator 12 of FIGURE 1. The rechargeable battery 23 is chargeable via inductive transcutaneous energy transfer. An electromagnetic coil 41 necessary to convert AC power into DC power, such as described in U.S. Patent No. 5,350,413, issued September 27, 1994 to Miller, is integrated into the housing 21 of the neurostimulator 12. In addition, charging circuitry 42 is coupled with the electromagnetic coil 41 and the rechargeable battery 23.

During charging, the patient 10 or other caregiver places an inductive charging wand over the implantation site of the neurostimulator 12. FIGURE 4 is a diagram showing an external charger 50 for use with the implantable neurostimulator 12 of FIGURE 1. The external charger 50 includes an inductive charging wand 51 that sends energy to the electromagnetic coil 14 through a transcutaneous inductive coupling, and the charging circuitry 42 converts the energy back into electrical current, which charges the onboard rechargeable battery 23. The inductive charging wand 51 encloses an inductive charging coil (not shown) on a distal end, which is placed over the implantation site of the neurostimulator 12. The inductive charging wand 51 is plugged into a wall outlet or other electrical power source with a power cord 54 or similar type of connector. Once placed over the implantation site, the patient 10 or caregiver presses a button 52 or other patient-operable control to start the charging of the neurostimulator 12.

The respective electromagnetic coils in the neurostimulator 12 and the inductive charging wand 51 form a transcutaneous inductive coupling when the button 52 is pressed, during which time alternating current energy is transferred into the neurostimulator 12, converted back into electrical current, and used to charge the rechargeable battery 23. Other ways for the neurostimulator 12 to transcutaneously receive energy from an external charging source are possible. For example, the neurostimulator 12 can include at least one photovoltaic cell, such as described in U.S. Patent Publication No. 2009/0326597, published December 31, 2009, abandoned. In one embodiment, the photovoltaic cell is subcutaneously placed away from the housing 21 of the neurostimulator 12. The photovoltaic cell is electrically interfaced with the charging circuitry 42 via electrical wiring. The photovoltaic cell absorbs light passing through the patient's translucent layers of skin, and converts the light into electrical energy, which is then fed through the charging circuitry 42 into the rechargeable battery 23. The photovoltaic cell can absorb either the light naturally coming from the patient's surroundings, or light generated by an energy generator containing a light source, such as described in U.S. Patent No. 6,961,619, issued November 01, 2005, to Casey. In one embodiment, the energy generator is placed against the patient's skin in alignment with the location of the photovoltaic cell. Still other ways to transcutaneously transmit energy are possible.

Ordinarily, the charging circuitry 42 charges the rechargeable battery 23, but prevents overcharging beyond the limits of the battery. In a further embodiment, the charging circuitry 42 measures the rechargeable battery's state of charge for reporting status to the patient 10 or caregiver. The charging circuitry 42 is electrically interfaced with the microprocessor controller, and provides the state of charge of the rechargeable battery 23. The neurostimulator 12 can provide feedback concerning the state of charge by transmitting status information to the external programmer or another remotely-interfaceable interrogation device using a transceiver and the antenna, either at the request of the external programmer or when the state of charge reaches a predetermined level. The external programmer can be the programming wand, as described *infra,* or be included as part of the charging circuitry 42. In a further embodiment, the neurostimulator can transmit status information to the patient-operable external controller. Other ways of interacting with the charging circuitry 42 are possible.

Referring back to FIGURE 2A, the neurostimulator 12 externally includes a header 24 to securely receive and connect to the therapy lead 13. In one embodiment, the header 24 encloses a receptacle 25 into which a single pin for the therapy lead 13 can be received, although two or more receptacles could also be provided, along with the requisite additional electronic circuitry 22. The header 24 internally includes a lead connector block (not shown) and a set of set screws 26.

In one embodiment, the housing 21 can also contain a heart rate sensor 31 that is electrically interfaced with the logic and control circuitry, which receives the patient's sensed heart rate or, alternatively, HRV, as sensory inputs. In a further embodiment, the heart rate sensor 31 is either external to or physically separate from the neurostimulator 12 proper, but is operatively coupled, either through physical wired connection or via wireless interface. The heart rate sensor 31 monitors heart rate or HRV using an ECG-type electrode. Through the electrode, the patient's heart beat can be sensed by detecting ventricular depolarization or similar physiology. In a further embodiment, a plurality of electrodes can be used to sense voltage differentials between electrode pairs, which can undergo signal processing for further cardiac physiological measures, for instance, detection of the P-wave, QRS complex, and T-wave. The heart rate sensor 31 provides the sensed heart rate or HRV to the control and logic circuitry as sensory inputs that can be used to determine the presence of arrhythmias, especially VT.

As mentioned above, the neurostimulator 12 can be interrogated preferably prior to implantation and throughout the therapeutic period with a healthcare provider-operable external programmer and programming wand (not shown) for checking proper operation, downloading recorded data, diagnosing problems, and programming operational parameters parameters and operating modes, such as described in commonly-assigned U.S. Patent applications, Serial No. 13/314,130 and 13/352,244, cited *supra.* In particular, the neurostimulator 12 is interrogated prior to starting of titration of VNS therapy. In one embodiment, the external programmer executes application software specifically designed to interrogate the neurostimulator 12. The programming computer interfaces to the programming wand through a standardized wired or wireless data connection. The programming wand can be adapted from a Model 201 Programming Wand, manufactured and sold by Cyberonics, Inc. and the application software can be adapted from the Model 250 Programming Software suite, licensed by Cyberonics, Inc. Other configurations and combinations of external programmer, programming wand and application software are possible.

The neurostimulator 12 delivers VNS under control of the electronic circuitry 22. The stored stimulation parameters are programmable. Each stimulation parameter can be independently programmed to define the characteristics of the cycles of therapeutic stimulation and inhibition to ensure optimal stimulation for a patient 10. The programmable stimulation parameters include output current, signal frequency, pulse width, signal ON time, signal OFF time, magnet activation (for VNS specifically triggered by a magnetic signal, such as during the autotitration process in one embodiment), and reset parameters. Other programmable parameters are possible. In addition, sets or "profiles" of pre-selected stimulation parameters can be provided to physicians with the external programmer and fine-tuned to a patient's physiological requirements prior to being programmed into the neurostimulator 12, such as described in commonly-assigned U.S. Patent application, entitled "Computer-Implemented System and Method for Selecting Therapy Profiles of Electrical Stimulation of Cervical Vagus Nerves for Treatment of Chronic Cardiac Dysfunction," Serial No. 13/314,138, filed on December 7, 2011, pending. In a further embodiment, sets or "profiles" of pre-selected stimulation parameters can be provided to physicians, which can be selected with the assistance of an external programmer and fine-tuned to a patient's physiological requirements prior to being programmed into the neurostimulator 12 following implantation.

Referring next to FIGURE 2B, the therapy lead 13 delivers an electrical signal from the neurostimulator 12 to the vagus nerve 15, 16 via the helical electrodes 14. On a proximal end, the therapy lead 13 has a lead connector 27 that transitions an insulated electrical lead body to a metal connector pin 28. During implantation, the connector pin 28 is guided through the receptacle 25 into the header 24 and securely fastened in place using the set screws 26 to electrically couple the therapy lead 13 to the neurostimulator 12. On a distal end, the therapy lead 13 terminates with the helical electrode 14, which bifurcates into anodic and cathodic electrodes. In one embodiment, the lead connector 27 is manufactured using silicone and the connector pin 28 is made of stainless steel, although other suitable materials could be used, as well. The insulated lead body 13 utilizes a silicone-insulated alloy conductor material.

Preferably, the helical electrodes 14 are placed over the cervical vagus nerve 15, 16 at the location below where the superior and inferior cardiac branches separate from the cervical vagus nerve. In alternative embodiments, the helical electrodes may be placed at a location above where one or both of the superior and inferior cardiac branches separate from the cervical vagus nerve. In one embodiment, the helical electrodes 14 are positioned around the patient's vagus nerve oriented with the end of the helical electrodes 14 facing the patient's head. In an alternate embodiment, the helical electrodes 14 are positioned around the patient's vagus nerve 15, 16 oriented with the end of the helical electrodes 14 facing the patient's heart 17. At the distal end, the insulated electrical lead body 13 is bifurcated into a pair of lead bodies that are connected to a pair of electrodes proper. The polarity of the electrodes could be configured into a monopolar cathode, a proximal anode and a distal cathode, or a proximal cathode and a distal anode.

The VNS is delivered as a multimodal set of therapeutic and event-based doses, which are system output behaviors that are pre-specified within the neurostimulator 12 through the stored stimulation parameters and timing cycles implemented in firmware programming and executed by the microprocessor controller. The selections of therapeutic dose and duty cycle are a tradeoff among competing medical considerations. FIGURE 3 is a graph 60 showing, by way of example, the relationship between the targeted therapeutic efficacy 63 and the extent of potential side effects 64 resulting from use of the implantable neurostimulator 12 of FIGURE 1. The x-axis represents the duty cycle 61. The *y*-axis represents relative quantified physiological response 62 to VNS therapy. The neurostimulation is delivered bi-directionally and at an intensity that is insufficient to elicit pathological or acute physiological side effects, such as acute cardiac arrhythmias, and without the requirement of an enabling physiological feature or triggering physiological marker, such as heart rate or HRV.

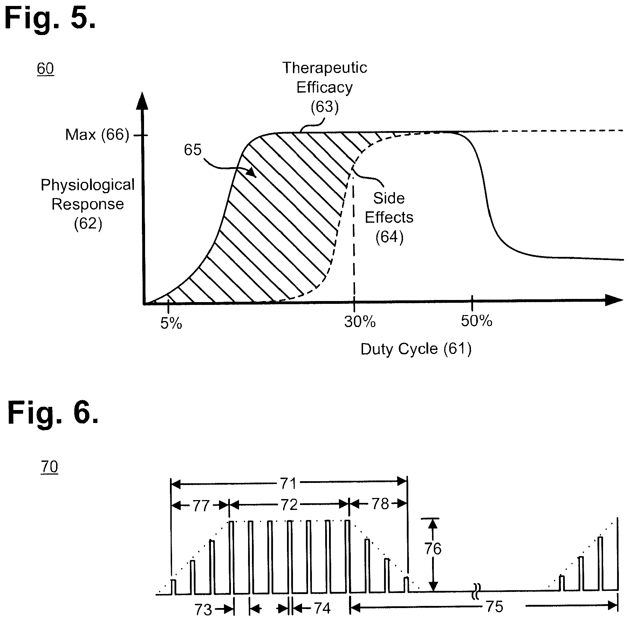

The duty cycle 61 is determined by dividing the stimulation ON time by the sum of the ON and OFF times of the neurostimulator 12 during a single ON-OFF cycle. In a further embodiment, the stimulation time can include ramp-up and ramp-down times respectively preceding and following the ON time during which the neurostimulator 12 delivers the VNS at full output current, such as further described *infra* with reference to FIGURE 6. Ramp-up and ramp-down times may be necessary, for instance, when the stimulation frequency exceeds a minimum threshold. The physiological response 62 can be expressed quantitatively for a given duty cycle 61 as a function of the targeted therapeutic efficacy 63 and the extent of potential side effects 64. The maximum level ("max") 66 of physiological response 62 signifies the highest point of targeted therapeutic efficacy 63 or potential side effects 64.

The therapeutic efficacy 63 represents the intended effectiveness of VNS in provoking a beneficial physiological response, which may be patient- or population-dependent. In contrast to conventional feedback-driven VNS approaches that require an enabling physiological feature to gauge stimuli delivery efficacy, the acute responses and chronic contributing factors need not be (and are likely not) directly observed contemporaneous to VNS delivery. Rather, the contributing factors could be clinically measured over time, such as in-clinic during patient follow up via ECG trace or other metric. As well, in on-going laboratory studies involving canines, increased heart beat regularity during VNS on time, as exhibited through decreased heart rate variability, has been creditably detected through 24-hour Holter monitoring during vagal neural stimulation at a constant continuously-cycling level below a subcardiac threshold, above which there is a slowing of heart rate, conduction velocity or other cardiac artifact.

The therapeutic efficacy 63 can be quantified by assigning values to the realized acute and chronic responses, which together contribute to and synergistically produce the beneficial physiological response. Acute responses include realized changes in HRV, increased coronary flow, reduction in cardiac workload through vasodilation, and improvement in left ventricular relaxation. Chronic factors include improved cardiovascular regulatory function, decreased negative cytokine production, increased baroreflex sensitivity, increased respiratory gas exchange efficiency, favorable gene expression, renin-angiotensin-aldosterone system down-regulation, anti-arrhythmic, anti-apoptotic, and ectopy-reducing anti-inflammatory effects. Still other acute responses and chronic factors are possible.

Beneficial physiological response is generally also considered to be patient dependent, whereby certain of the contributing factors may be more important for one patient as compared to other patients, and no single contributing factor is fully dispositive or conclusive of whether the physiological response is beneficial for any given patient. The contributing factors can be combined in any manner to quantify the relative level of therapeutic efficacy 63, including weighting particular factors more heavily than others, by tailoring the importance of each contributing factor on a patient-specific or population-specific manner, or applying statistical or numeric functions based directly on or derived from realized changes to the patient's physiology. For example, therapeutic goals of achieving an increase of HRV of 10% and decreased negative cytokine production of 3% may be desired for a particular patient with equal weight assigned to each of these goals. Maximum physiological response 66 occurs when these goals are substantially met.

Empirically, therapeutic efficacy 63 steeply increases beginning at around a 5% duty cycle (patient-dependent), as beneficial physiological response 62 is realized, and levels off in a plateau near the maximum level 66 of physiological response 62 at around a 30% duty cycle (patient-dependent), although some patients may require higher (or lower) duty cycles to show similar beneficial physiological response 62. Thereafter, therapeutic efficacy 63 begins decreasing at around a 50% duty cycle (patient-dependent) and continues in a plateau near a 25% physiological response (patient-dependent) through the maximum 100% duty cycle.

The physiological response and occurrence of side effects to different combinations of VNS parameters and timing cycles has been empirically evaluated in pre-clinical work on canines. Like the therapeutic efficacy 63, side effects 64 may be patient- or population-dependent and can be quantified by assigning values to the realized acute and chronic side effects. For example, benign acute side effects, such as coughing, could be assigned a low value, while pathological side effects, like bradycardia, could be rated significantly higher to reflect level of severity. In the canine study, the only side effects that were observed were coughing and throat irritation, retching, and bradycardia during 4-6 weeks of gradual titration. Following titration, the only remaining side effect that was observed was bradycardia, which was observed in the following ranges:
- Right VNS: In an awake animal, bradycardia was typically observed beginning at approximately 1.25-1.5 mA at a pulse width of 500 µsec and approximately 1.5-1.75 mA at a pulse width 250 µsec, independent of stimulation frequency. However, the magnitude of the current-dependent bradycardia increased with stimulation frequency, with the highest level of bradycardia observed at 20 Hz, the lowest level observed at 10 Hz, and an intermediate level observed at 15 Hz. During sleep, bradycardia was observed at similar parameters as in an awake animal.
- Left VNS: In an awake animal, bradycardia was only observed at amplitudes greater than 2.5 mA, and primarily in the 3.0-3.5 mA range. Stimulation frequencies of 15 or 20 Hz were more likely to produce bradycardia at the lower end of that amplitude range, and a stimulation frequency of 10 Hz was more likely to produce bradycardia only at the upper end of the range. In some animals, no bradycardia was observed at any combination of parameters up to the maximum stimulation current of 3.5 mA.

During sleep, bradycardia was observed at similar parameters as in an awake animal. Comparable ranges of side effects in humans are expected.

In the absence of patient physiology of possible medical concern, such as acute cardiac arrhythmias, VNS is delivered in therapeutic doses that each use alternating cycles of stimuli application (ON) and stimuli inhibition (OFF) that are tuned to activate both afferent and efferent pathways. Stimulation results in parasympathetic activation and sympathetic inhibition, both through centrally-mediated pathways and through efferent activation of preganglionic neurons and local circuit neurons. FIGURE 6 is a timing diagram showing, by way of example, a stimulation cycle and an inhibition cycle of VNS 70 as provided by implantable neurostimulator 12 of FIGURE 1. The stimulation parameters enable the electrical stimulation pulse output by the neurostimulator 12 to be varied by both amplitude (output current 66) and duration (pulse width 64). The set of stimulation parameters and timing cycle used depends upon the operating mode of therapy desired, which in the described embodiment is the titration mode further described below with reference to FIGURES 7 and 8. Other modes are possible. The number of output pulses delivered per second determines the signal frequency 63. In one embodiment, a pulse width in the range of 100 to 250 µsec is used to deliver between 0.1 and 10 mA of output current at a signal frequency of 5 to 20 Hz, although other therapeutic values could be used as appropriate.

In the simplest case, the stimulation time equals the time period during which the neurostimulator 12 is ON and delivering pulses of stimulation. The OFF time 75 equals the time period occurring in-between stimulation times 71 during which the neurostimulator 12 is OFF and inhibited from delivering stimulation. In one embodiment, the neurostimulator 12 implements either or both of a ramp-up time 77 and a ramp-down time 78 that respectively precede and follow the ON time 72 during which the neurostimulator 12 is ON and delivering pulses of stimulation at the full output current 76. The ramp-up time 77 and ramp-down time 78 are used when the stimulation frequency is at least 10 Hz, although other minimum thresholds could be used, and both ramp-up and ramp-down times 77, 78 last two seconds, although other time periods could also be used. The ramp-up time 77 and ramp-down time 78 allow the strength of the output current 76 of each output pulse to be gradually increased and decreased, thereby avoiding deleterious reflex behavior due to sudden delivery or inhibition of stimulation at a full-strength programmed level of intensity.

As described above, titrating VNS stimulation by having a physician perform each step in the titration creates a medically unnecessary delay in delivering VNS at a full therapeutic intensity to the patient 10. On the other hand, allowing a patient 10 to autotitrate the VNS intensity without physician oversight creates a safety concern. A patient may titrate more aggressively than the physician would like, or may experience painful stimulation if the titration is not performed correctly. Allowing the patient 10 to titrate the VNS stimulation subject to limits set by the patient's physician allows both to avoid the delays associated with visiting the physician's office and to mitigate the safety issues associated with an unsupervised autotitration. FIGURE 7 is a flow diagram showing an implantable neurostimulator-implemented method 80 for treatment of chronic cardiac dysfunction with bounded titration, said method is not according to the invention and present for illustration purposes only.

Initially, after a patient 10 recovers from the implantation of the neurostimulator, a healthcare professional programs the neurostimulator 12 using the external programmer and stores the autotitration operating mode into the recordable memory 29 of the neurostimulator (step 81). As mentioned *supra,* the operating mode includes one or more parameters defining the highest-intensity dose of VNS stimulation that the patient 10 can command the neurostimulator 12 to deliver during the autotitration process ("maximum stimulation intensity "). This maximum stimulation intensity can be defined either through a single parameter, such as the output current, or through a combination of parameters, with the parameters defining the maximum charge delivered. The autotitration operating mode can include other limitations on the autotiration process, such as a predefined schedule according to which the patient 10 can up-titrate the VNS stimulation. For example, the operating mode can specify that the VNS therapy can be up-titrated once a week for a period of thirty days. The autotitration operating mode can further specify a routine for adjusting the level of VNS stimulation delivered based on the feedback received from the patient 10 regarding whether the up-titrated VNS is tolerable, as further described with reference to FIGURE 8. In addition, the operating mode includes the parameters defining an initial dose of VNS stimulation delivered at the start of the autotitration ("titration dose"). In one embodiment, the parameters of the titration dose can be an output current of 0.25 mA, pulse width of 250 µsec, signal frequency of 10 Hz, On time of 14 seconds, and Off time of 1.1 minutes. The intensity of the titration dose is parametrically defined to not exceed the maximum stimulation intensity, which includes the titration dose having one or more of an output current lower or equal to the maximum stimulation intensity output current, a duty cycle lower or equal to the maximum stimulation intensity duty cycle, a frequency lower or equal to the maximum stimulation intensity frequency, and a pulse width shorter or equal to the maximum stimulation intensity pulse width. Finally, the autotitration operating mode is further configured to suspend VNS upon receiving an appropriate signal from the external controller.

Once the autotitration operating mode is received (step 91), the neurostimulator 12 starts therapeutically delivering the titration dose of VNS stimulation (step 82). When the patient 10 uses the external controller to up-titrate the intensity, the neurostimulator 12 receives from the external controller a unique signal, such as a magnetic signal, associated with the command to up-titrate the intensity of the titration dose (step 83). For example, if the patient-operable external controller is an external magnet, the signal can be a unique predefined patter of swipes with the magnet performed by the patient 10 when the patient desires to up-titrate the delivered VNS intensity. Similarly, if the patient-operable external controller is an external electromagnetic controller, such as described in the 13/352,244 application cited *supra,* the patient 10 can press an appropriately-labeled button on the electromagnetic controller when the patient 10 desires to up-titrate the VNS therapy, with the electromagnetic controller sending to the neurostimulator a unique magnetic signal associated with the command to increase the intensity of the titration dose.

Upon receipt of the signal (step 83), the neurostimulator 12 determines whether further increasing the intensity at which the titration dose is delivered would exceed the limits set down by the set down in the autotitration operating mode, and if the limits would be exceeded (step 84), the therapeutic delivery of the titration dose continues at the same intensity as before (step 85), ending the method 80. For example, if increasing the intensity of the titration dose would make the titration dose intensity higher than the maximum intensity dose, the titration dose intensity would not be increased, but would continue at the same level. At that point, the only way for the intensity of the delivered VNS therapy to be increased is for the patient to visit a physician, who will reprogram the neurostimulator 12 with a set of updated limits. For example, if maximum intensity dose is initially defined as having an output current of 1.0 mA, upon autotitrating the intensity once a week for 4 weeks, the output current at which the titration dose is delivered would reach 1.0 mA, requiring the patient to visit the physician's office, where the physician would increase the maximum intensity to, for example, 2.0 mA. After this follow-up visit, the patient could continue autotitrating the VNS therapy until the updated limits are reached.

Similarly, in a further embodiment, if the patient 10 attempts to increase the intensity of the titration dose contrary to other limits, such contrary to the predefined schedule, the neurostimulator 12 will continue delivering the titration dose without increasing the intensity (step 85).

If at step 84 the intensity of the titration dose would not exceed the limits, the intensity of the titration dose delivered to the patient is adjusted as specified in the autotitration operating mode (step 96), such as by increasing one or more parameters defining the titration dose intensity, as further described in detail with reference to FIGURE 8. The delivery of the titration dose at the adjusted intensity continues per step 82. While the embodiment described in reference to FIGURE 8 describes the autotitration being performed after an implantation (or re-implantation) of the neurostimulator 12, in a further embodiment, the method 80 can be performed under other circumstances.

While physician-imposed limits on autotitration are instrumental in mitigating safety concerns, receiving patient feedback regarding whether an increased intensity of stimulation is tolerable to the patient 10 provides additional safeguards against a patient 10 autotitrating VNS stimulation overly aggressively. Furthermore, adjusting VNS intensity based on the patient feedback presents an alternative to suspending VNS stimulation altogether upon the increased level of stimulation becoming intolerable, thus preserving the continuity of the titration. FIGURE 8 is a flow diagram showing a routine 90, not according to the invention and present for illustration purposes only, for adjusting intensity of VNS therapy during autotitration based on patient feedback for the method 80 of FIGURE 7. Continuing with the example of FIGURE 8, the initial intensity of the titration dose can be defined using the following parameters: an output current of 0.25 mA, pulse width of 250 µsec, signal frequency of 10 Hz, ON time of 14 seconds, and OFF time of 1.1 minutes. Once the neurostimulator 12 receives the unique signal from the patient-operable external controller associated with a command to up-titrate the titration dose intensity, the neurostimulator 12 increases one of the parameters defining the titration dose intensity, with the titration dose being continuously delivered upon the intensity being adjusted per step 82 (step 91). In the described embodiment, the parameter increased is output current (step 91), with the increase being 0.25 mA. Following the increase of the delivered intensity of the titration dose, the neurostimulator 12 receives feedback from the patient-operable external controller regarding whether the increased intensity titration dose is subjectively tolerable to the patient 10 (step 92). The feedback is received as one or more unique signals from the controller, with differing signals indicating whether the increased intensity of the titration dose is tolerable or not. For example, if the external controller is an external magnet, the patient 10 can indicate whether the increased intensity is tolerable or not by performing different patterns of swipes with the magnet. Similarly, if the patient-operable external controller is an external electromagnetic controller, the controller can transmit unique magnetic signals to the neurostimulator 12 that are associated with the increased intensity being tolerable or intolerable.

If the increased intensity is tolerable to the patient (step 93), the routine 90 returns to step 91, and the output current of the delivered titration dose is further increased (step 91). If the increased intensity is intolerable to the patient (step 103), the output current is reverted to the level that the patient has indicated tolerable, or if the patient has not indicated a tolerable level, to the initial level indicated in the autotitration operating mode (step 94). In the same step, a different parameter of the delivered titration dose, such as pulse width, is increased, with the pulse width increasing to 500 µsec in the described embodiment (step 94). Patient feedback is received regarding whether the titration dose delivered at the increased pulse width is tolerable (step 95), and if the patient 10 indicates that the titration dose at the increased pulse width is tolerable (step 96), another parameter, such as signal frequency, is also increased, with the frequency being increased to 15 Hz in the described embodiment (step 97). If the patient 10 indicates that titration dose delivered at the increased pulse width is intolerable (step 96), the pulse width is reverted to the initial setting and another parameter, such as signal frequency, is increased, with the signal frequency being increased to 15 Hz in the described embodiment (98).

Upon the increase in signal frequency in steps 97 or 98, patient feedback is received regarding whether the increase in frequency is tolerable (step 99). If the delivery of the increase is not tolerable (step 100), the signal frequency is reverted back to the initial level of 10 Hz (step 101), ending the routine 90. Until the next signal associated with a command to up-titrate the VNS therapy is received from the external controller, the titration dose is delivered at the intensity indicated as tolerable by the patient, or if the patient 10 has indicated that the increased intensity is not tolerable, at the initial titration dose intensity. If the delivery of the titration dose at the increased signal frequency is tolerable (step 100), the frequency is further increased to, in the described embodiment, 20 Hz (step 102). Patient feedback is once again received regarding whether the delivery of the titration dose at the further increased signal frequency is tolerable (step 103), and if the titration dose is tolerable (104), the routine 90 terminates, with the neurostimulator 12 continuing to deliver the increased titration dose per step 82 above. If the titration dose delivered at the further increased signal frequency is not tolerable (step 104), the signal frequency is reverted to a level indicated by the patient as tolerable (step 105), ending the routine. Other levels of the parameters can be used in the adjustment of the titration dose intensity. While the described embodiment, multiple parameters are adjusted, in a further embodiment, only a single parameter, such output current, can be adjusted in the routine 90. Furthermore, as described *supra,* at any moment in the routine 100, the patient 10 can indefinitely suspend VNS therapy using the external controller.

While the invention has been particularly shown and described as referenced to the embodiments thereof, those skilled in the art will understand that the foregoing and other changes in form and detail may be made therein without departing from the scope.

## Claims

1. A system for providing electrical stimulation of a cervical vagus nerve (15, 16) for treatment of
chronic cardiac dysfunction with bounded titration, comprising:
a patient-operable external controller configured to transmit a plurality of unique signals comprising a unique signal associated with an up-titration command;
a pair of helical electrodes (14);
a nerve stimulation lead (13); and
an implantable neurostimulator (12) comprising:
a pulse generator configured to deliver electrical therapeutic stimulation tuned to restore autonomic balance through continuously-cycling, intermittent and periodic electrical pulses simultaneously delivered at an intensity that avoids acute physiological side effects and with an unchanging cycle not triggered by physiological markers, said electrical therapeutic stimulation is configured to be delivered in a manner that results in creation and propagation, in particular,in both afferent and efferent directions, of action potentials within neuronal fibers comprising said cervical vagus nerve of a patient through the pair of helical electrodes via the electrically coupled nerve stimulation therapy lead; and
a recordable memory (29) storing an autotitration operating mode comprising a maximum stimulation intensity and configured to increase an intensity of the delivered electrical therapeutic stimulation up to a level not exceeding the maximum stimulation intensity upon receipt of the unique signal associated with the up-titration command.

2. A system according to Claim 1, further comprising:
an external programmer operable by a healthcare professional and configured to store the autotitration operating mode into the recordable memory.

3. A system according to Claim 1 or 2, wherein the plurality of the unique signals further comprises at least one of a unique signal indicating that the increased intensity of the electrical therapeutic stimulation is tolerable to the patient and a unique signal indicating that the increased intensity of the electrical therapeutic stimulation is not tolerable to the patient.

4. A system according to one of the preceding claims , wherein the autotitration operating mode is further configured to increase the intensity of the delivered therapeutic stimulation when the unique signal associated with the up-titration command is received in accordance with a predefined schedule comprised in the autotitration operating mode.

5. A system according to one of the preceding claims, further comprising:
a magnetic switch configured to control the pulse generator in response to receipt of one of the plurality of the unique signals,
wherein the unique signals are magnetic signals.

6. A system according to one of the preceding claims, further comprising:
a rechargeable battery comprised in the neurostimulator and configured to power the neurostimulator; and
an energy receiver configured to transcutaneously receive energy and recharge the rechargeable battery with the received energy.

7. A system according to claim 1;
wherein the recordable memory is configured to perform a stimulation autotitration following at least one of the neurostimulator's implantation into the patient and a disruption of the delivery of the electrical therapeutic stimulation caused by a depletion of the neurostimulator's power; and further comprising
an energy receiver configured to transcutaneously receive energy and recharge the rechargeable battery with the received energy.

8. A system according to Claim 7, further comprising:
an external energy generator configured to transcutaneously transmit the energy to the energy receiver.

9. A system according to Claim 7 or 8, further comprising:
a first electromagnetic coil comprised in the energy receiver; and
a second electromagnetic coil comprised in the external energy generator.

10. A system according to Claim 7, 8 or 9, further comprising:
a photovoltaic cell comprised in the energy receiver; and
a light source comprised in the external energy generator.

11. A system according to Claim 7, 8, 9 or 10, further comprising:
a state of charge gauge configured to measure the rechargeable battery's state of charge.

12. A system according to Claim 11, further comprising at least one of:
a patient feedback circuit configured to notify a user when the state of charge reaches a predetermined level.

13. A system according to Claim 12, wherein the patient feedback circuit further configured to provide the state of charge in response to a request by an external programmer.

## Patentansprüche

1. Ein System für eine elektrische Stimulation eines Zervikalnervs (15, 16) zur Behandlung einer chronischen kardialen Dysfunktion mit limitierter Dosis, das umfasst:
einen von einem Patienten bedienbaren externen Kontroller, der dazu ausgebildet ist,
mehrere eindeutige Signale, die ein Signal umfassen, das mit einer Höherdosierung-Anweisung assoziiert ist, zu übermitteln;
ein Paar helischer Elektroden (14);
ein Nervstimulationskabel (13); und
einen implantierbaren Neurostimulator (12) der umfasst:
einen Pulsgenerator, der dazu ausgebildet ist, eine elektrische therapeutische Stimulation zu liefern, die darauf abgestimmt ist, ein autonomes Gleichgewicht durch kontinuierlichzyklisch, intermittierend und periodische elektrische Pulse wiederherzustellen, die gleichzeitig mit einer Intensität, die akute physiologische Nebenwirkungen vermeidet, und mit einem unveränderlichen Zyklus, der nicht durch physiologische Marker getriggert wird, geliefert werden, wobei die genannte elektrische therapeutische Stimulation dazu ausgebildet ist, in einer Weise geliefert zu werden, die in der Erzeugung und Propagation von Aktionspotentialen, insbesondere in sowohl afferente als auch efferente Richtung, innerhalb neuronaler Fasern, die den genannten Zervikalnerv eines Patienten umfassen, durch das Paar helischer Elektroden über das elektrisch verbundene Nervstimulationskabel resultieren; und
einen beschreibbaren Speicher (29), der einen Autodosiermodus speichert, der eine maximale Stimulationsintensität umfasst und dazu ausgebildet ist, eine Intensität der gelieferten elektrischen therapeutischen Stimulation bei Empfang des eindeutigen Signals, das mit der Höherdosierung-Anweisung assoziiert ist, bis zu einem Niveau zu erhöhen, das die maximale Stimulationsintensität nicht überschreitet.

2. Ein System gemäß Anspruch 1, das weiterhin umfasst:
einen externen Programmierer, der von einem professionellen medizinischen Personal bedient werden kann und dazu ausgebildet ist, den Autodosiermodus in den beschreibbaren Speicher zu speichern.

3. Ein System gemäß Anspruch 1 oder 2, in dem die mehreren eindeutigen Signale weiterhin zumindest eines von einem eindeutigen Signal, das anzeigt, dass die erhöhte Intensität der elektrischen therapeutischen Stimulation von dem Patienten toleriert wird, und einem eindeutigen Signal, das anzeigt, dass die erhöhte Intensität der elektrischen therapeutischen Stimulation von dem Patienten nicht toleriert wird, umfasst.

4. Ein System gemäß einem der vorhergehenden Ansprüche, in dem der Autodosiermodus weiterhin dazu ausgebildet ist, die Intensität der gelieferten therapeutischen Stimulation zu erhöhen, wenn das eindeutige Signal, das mit der Höherdosierung-Anweisung assoziiert ist, in Übereinstimmung mit einem vordefinierten Zeitplan, der in dem Autodosiermodus enthalten ist, empfangen wird.

5. Ein System gemäß einem der vorhergehenden Ansprüche, das weiterhin umfasst:
einen Magnetschalter, der dazu ausgebildet ist, den Pulsgenerator in Reaktion auf das Empfangen eines der mehreren eindeutigen Signale zu steuern,
wobei die eindeutigen Signale magnetische Signale sind.

6. Ein System gemäß einem der vorhergehenden Ansprüche, das weiterhin umfasst:
eine wiederaufladbare Batterie, die in dem Neurostimulator enthalten ist und dazu ausgebildet ist, den Neurostimulator mit Energie zu versorgen; und
einen Energieempfänger, der dazu ausgebildet ist, subkutan Energie zu empfangen und die wiederaufladbare Batterie mit der empfangenen Energie wieder aufzuladen.

7. Ein System gemäß Anspruch 1,
in dem der beschreibbare Speicher dazu ausgebildet ist, nachfolgend auf zumindest eines von der Implantation des Neurostimulators in den Patienten und einer Unterbrechung der Lieferung der elektrischen therapeutischen Stimulation aufgrund einer Erschöpfung der Energie des Neurostimulators eine Stimulations-Autodosierung auszuführen; und weiterhin umfassend
einen Energieempfänger, der dazu ausgebildet ist, transkutan Energie zu empfangen und die wiederaufladbare Batterie mit der empfangenen Energie wieder aufzuladen.

8. Ein System gemäß Anspruch 7, das weiterhin umfasst:
einen externen Energiegenerator, der dazu ausgebildet ist, die Energie transkutan an den Energieempfänger zu liefern.

9. Ein System gemäß Anspruch 7 oder 8, das weiterhin umfasst:
eine erste elektromagnetische Spule, die in dem Energieempfänger enthalten; und
eine zweite elektromagnetische Spule, die in dem externen Energiegenerator enthalten ist.

10. Ein System gemäß Anspruch 7, 8 oder 9, das weiterhin umfasst:
eine Photovoltaikzelle, die in dem Energieempfänger enthalten ist; und
eine Lichtquelle, die in dem externen Energiegenerator enthalten ist.

11. Ein System gemäß Anspruch 7, 8, 9 oder 10, das weiterhin einen Ladungszustandsmesser umfasst, der dazu ausgebildet ist, den Ladungszustand der wiederaufladbaren Batterie zu messen.

12. Ein System gemäß Anspruch 12, das weiterhin zumindest eine Patient-Feedback-Schaltung umfasst, die dazu ausgebildet ist, einen Nutzer zu benachrichtigen, wenn der Ladungszustand ein vorbestimmtes Niveau erreicht.

13. Ein System gemäß Anspruch 12, in dem die Patient-Feedback-Schaltung weiterhin dazu ausgebildet ist, in Reaktion auf eine Anforderung durch einen externen Programmierer den Ladungszustand anzugeben.

## Revendications

1. Système de fourniture d'une stimulation électrique d'un nerf vagal cervical (15, 16) pour le traitement du dysfonctionnement cardiaque chronique avec titrage limité, comprenant :
un dispositif de commande externe pouvant être actionné par un patient conçu pour transmettre une pluralité de signaux uniques comprenant un signal unique associé à une commande de titrage à la hausse ;
une paire d'électrodes hélicoïdales (14) ;
un fil de stimulation de nerf (13) ; et
un neurostimulateur implantable (12) comprenant :
un générateur d'impulsions conçu pour administrer une stimulation thérapeutique électrique ajustée pour restaurer l'équilibre autonome à travers le cyclage continuel, des impulsions électriques intermittentes et périodiques administrées simultanément à une intensité qui évite les effets secondaires physiologiques aigus et avec un cycle non changeant non déclenché par des marqueurs physiologiques, ladite stimulation thérapeutique électrique étant conçue pour être administrée d'une manière qui résulte en création et propagation, en particulier, dans à la fois des sens afférents et efférents, de potentiels d'action à l'intérieur des fibres neuronales comprenant ledit nerf vagal cervical d'un patient à travers la paire d'électrodes hélicoïdales par l'intermédiaire du fil de thérapie de stimulation de nerf électriquement couplé ; et
une mémoire enregistrable (29) stockant un mode de fonctionnement d'autotitrage comprenant une intensité de stimulation maximale et conçue pour accroître une intensité de la stimulation thérapeutique électrique administrée jusqu'à un niveau n'excédant pas l'intensité de stimulation maximale lors de la réception du signal unique associé à la commande de titrage à la hausse.

2. Système selon la revendication 1, comprenant en outre :
un programmeur externe pouvant être actionné par un professionnel de soin de santé et conçu pour stocker le mode de fonctionnement d'autotitrage dans la mémoire enregistrable.

3. Système selon la revendication 1 ou 2, la pluralité des signaux uniques comprenant en outre au moins l'un d'un signal unique indiquant que l'intensité accrue de la stimulation thérapeutique électrique est tolérable pour le patient et d'un signal unique indiquant que l'intensité accrue de la stimulation thérapeutique électrique n'est pas tolérable pour le patient.

4. Système selon l'une des revendications précédentes, le mode de fonctionnement d'autotitrage étant en outre conçu pour accroître l'intensité de la stimulation thérapeutique administrée lorsque le signal unique associé à la commande de titrage à la hausse est reçu en fonction d'un programme prédéfini compris dans le mode de fonctionnement d'autotitrage.

5. Système selon l'une des revendications précédentes, comprenant en outre :
un commutateur magnétique conçu pour commander le générateur d'impulsions en réponse à la réception d'un de la pluralité des signaux uniques,
les signaux uniques étant des signaux magnétiques.

6. Système selon l'une des revendications précédentes, comprenant en outre :
une batterie rechargeable comprise dans le neurostimulateur et conçue pour alimenter le neurostimulateur ; et
un récepteur d'énergie conçu pour recevoir de manière transcutanée de l'énergie et recharger la batterie rechargeable avec l'énergie reçue.

7. Système selon la revendication 1 :
la mémoire enregistrable étant conçue pour effectuer un autotitrage de stimulation suite au moins à l'une de l'implantation du neurostimulateur chez le patient et d'une rupture d'administration de la stimulation thérapeutique électrique causée par un épuisement de l'énergie du neurostimulateur ; et comprenant en outre
un récepteur d'énergie conçu pour recevoir de manière transcutanée de l'énergie et recharger la batterie rechargeable avec l'énergie reçue.

8. Système selon la revendication 7, comprenant en outre :
un générateur d'énergie externe conçu pour transmettre de manière transcutanée l'énergie au récepteur d'énergie.

9. Système selon la revendication 7 ou 8, comprenant en outre :
une première bobine électromagnétique comprise dans le récepteur d'énergie ; et
une seconde bobine électromagnétique comprise dans le générateur d'énergie externe.

10. Système selon la revendication 7, 8 ou 9, comprenant en outre :
une cellule photovoltaïque comprise dans le récepteur d'énergie ; et
une source de lumière comprise dans le générateur d'énergie externe.

11. Système selon la revendication 7, 8, 9 ou 10, comprenant en outre :
un état de jauge de charge conçu pour mesurer l'état de charge de la batterie rechargeable.

12. Système selon la revendication 11, comprenant en outre au moins l'un :
d'un circuit de rétroaction de patient conçu pour alerter un utilisateur lorsque l'état de charge atteint un niveau prédéterminé.

13. Système selon la revendication 12, le circuit de rétroaction de patient étant en outre conçu pour fournir l'état de charge en réponse à une requête par un programmeur externe.
